# EUROPEAN PATENT APPLICATION

(11) **EP 4 306 133 A1**
(43) Date of publication of application: **17.01.2024**
(21) Application number: 22767463.7
(22) Date of filing: 08.03.2022
(51) Int. Cl.: A61K 48/00, A61K 47/60, A61K 47/58, A61K 47/54, A61K 47/69, A61K 9/00, A61K 31/713, A61K 31/7088

(54) **COMPOSITION FOR ADMINISTRATION OF DOUBLE-STRANDED OLIGONUCLEOTIDE STRUCTURES USING ULTRASONIC NEBULIZER FOR PREVENTION OR TREATMENT OF RESPIRATORY VIRAL INFECTION INCLUDING COVID-19, PULMONARY FIBROSIS CAUSED BY VIRAL INFECTION, OR RESPIRATORY DISEASES**

(30) Priority: 08.03.2021 KR 20210029927
(71) Applicant: Bioneer Corporation, Daejeon 34302 (KR); Sirnagen Therapeutics Corporation, Daejeon 34302 (KR)
(72) Inventor: PARK, Han-Oh, Sejong 30151 (KR); LEE, Sang-Kyu, Daejeon 34016 (KR); YUN, Sung-Il, Daejeon 34022 (KR); KWON, Oh Seung, Sejong 30126 (KR); GOH, Eun-Ah, Sejong 30150 (KR); GOH, Young-Ho, Daejeon 35211 (KR); PARK, Jun-Hong, Daejeon 34538 (KR); SONG, Kang, Daejeon 34401 (KR); KIM, Jangseon, Daejeon 34011 (KR); LEE, Mi-Sun, Daejeon 34022 (KR); CHOI, Soon-Ja, Daejeon 34048 (KR)
(74) Representative: Mewburn Ellis LLP
(86) International application number: PCT/KR2022/003245
(87) International publication number: WO 2022/191567

(57) **Abstract**

The present invention relates to a composition for administering a double-stranded oligonucleotide structure using an ultrasonic nebulizer. According to the method, the double-stranded oligonucleotide according to the present invention forms self-assembled nanoparticles, which are 90 nm in size and have a neutral charge, and it is possible to deliver the double-stranded oligonucleotide specifically to the nasal cavity and lungs while maintaining not only the same concentration, molecular weight, purity, nanoparticle size, and osmolality as those of the stock material but also the target gene inhibitory activity without cytotoxicity. Thus, the present invention may be useful for the prevention or treatment of respiratory viral infections including COVID-19, pulmonary fibrosis caused by viral infection, or respiratory diseases.

## Description

### Technical Field

The present invention relates to a composition for administering a double-stranded oligonucleotide structure using an ultrasonic nebulizer, and in particular, to a composition for administering a double-stranded oligonucleotide structure, which is used for the prevention or treatment of respiratory viral infections including COVID-19, pulmonary fibrosis caused by viral infection, or respiratory disease, by using an ultrasonic nebulizer.

### Background Art

Drug delivery by inhalation is a drug delivery method that is easy to use, enables the drug to exhibit a desired effect even at a low drug dose, may avoid or minimize systemic side effects by allowing the drug to directly reach a target organ, especially the bronchi or lungs, and enables the drug to exhibit rapid effects. Inhalers are classified according to drug form into a metered-dose inhaler (MDI), a dry powder inhaler (DPI), and a nebulizer. The metered-dose inhaler is a device that releases a certain amount of a drug upon use by the pressure of the gas filled into a drug container, and the dry powder inhaler is not filled with any gas and is a form of inhaling a drug by the inhalation ability thereof. The nebulizer is a device that uses mechanical vibration to atomize a drug into small liquid particles, and then deliver the atomized particles into a patient through an inhalation tube by air pressure. Since the nebulizer delivers finely atomized drug particles whose effect is well delivered to the bronchi and alveoli, it has the advantage of being able to be used for respiratory viral infection, interstitial pulmonary fibrosis, chronic obstructive pulmonary disease, asthma, bronchiectasis, bronchitis, pneumonia, emphysema, and the like. Nebulizers may be classified according to atomization method into a compressor type nebulizer and an ultrasonic nebulizer. In the compressor-type nebulizer, the compressor creates atomized particles by passing air through the nebulizer drug chamber, and an aerosol is generated through the micropores of the vibrating mesh. In the ultrasonic nebulizer, ultrasonic waves vibrate the drug while passing through the drug chamber, thereby generating an aerosol. Depending on the equipment, an aerosol with a certain size is generated by passing air or microwaves through the micropores of the mesh.

Various attempts have been made to develop siRNA-based drugs as inhalants. However, these inhalants are mainly dry powder formulations, including hyaluronic acid-coated liposomal spray formulations (freeze-dried formulations), nanoparticle formulations comprising a mixture of albumin and PLGA (poly-lactic-co-glycolic acid), chitosan polymer formulations, cationic lipid or liposome formulations, and nanoparticle formulations comprising cell-penetrating proteins (MPG, TAT, CADY, LAH4). The biggest obstacle to the development of inhalants for liposome-based siRNA drugs is a phenomenon that the stability of liposomes breaks down breaks down after nebulization, such as fragmentation of liposomes after nebulization. To improve the stability of liposomes after nebulization, various formulation methods have been attempted, such as mixing liposomes with cholesterol, high-phase transition phospholipids, etc., or PEGylation of liposomes, but there is still a need to improve the stability of liposomes themselves and the drug stability in liposomes (Mindaugas Rudokas, Med. Princ. Pract. 2016 March; 25:60). In addition, in the case of cationic lipid or liposome formulations, particles themselves have a toxicity risk, and in the case of conventional RNAi-based drugs, they have a risk of causing toxicity by inducing innate immune responses to non-specifically activate pro-inflammatory cytokine and interferon responses, and show limited delivery efficiency and drug effects (Hasan Uludag, front. Bioeng. Biotechnol. 2020 July; 8:916).

In 1995, Guo and Kemphues reported that not only sense RNA but also antisense RNA is effective in inhibiting gene expression in *C. elegans,* and since then, studies have been conducted to identify the cause thereof. In 1998, Fire et al. first described the phenomenon in which injection of double-stranded RNA (dsRNA) inhibits gene expression by specifically degrading the mRNA corresponding thereto. This phenomenon was named RNA interference (RNAi). RNAi, a process that is used to inhibit gene expression, may exhibit a distinct effect of inhibiting gene expression in a simple manner at low cost, and thus the application range of this technology has expanded.

Since this technology of inhibiting gene expression may regulate the expression of a specific gene, it may remove a specific gene related to cancer, genetic disease or the like at the mRNA level, and may be used as an important tool for the development of therapeutic agents for disease treatment and validation of targets. As conventional techniques for inhibiting target gene expression, techniques of introducing a transgene for a target gene have been disclosed. These techniques include a method of introducing a transgene in the antisense direction with respect to the promoter and a method of introducing a transgene in the antisense direction with respect to the promoter.

Such RNA therapy targeting RNA is a method of removing the function of the gene of interest using oligonucleotides against the target RNA, and may be considered different from conventional methods in which therapeutic agents such as antibodies and small molecules mainly target proteins. Approaches for targeting RNA are roughly classified into two types: double-stranded-RNA mediated RNAi, and an antisense oligonucleotide (ASO). Currently, clinical trials are being attempted by targeting RNA in various diseases.

An antisense oligonucleotide (hereinafter referred to as "ASO") is short synthetic DNA designed to bind to a target gene according to Watson-Crick base pairing, and may specifically inhibit the expression of a specific nucleotide sequence of a gene. Thus, the antisense oligonucleotide has been used to study the roles of genes and to develop therapeutic agents capable of treating diseases such as cancer at the molecular level. These ASOs have the advantage of being able to be easily produced by setting various targets for inhibiting gene expression, and studies have been conducted on the use of ASOs in order to inhibit oncogene expression and cancer cell growth. A process of inhibiting the expression of a specific gene by the ASO is accomplished either by binding the ASO to a complementary mRNA sequence to induce RNase H activity and remove the mRNA or by interfering with the formation and progression of a ribosome complex for protein translation. In addition, it has been reported that the ASO binds to genomic DNA to form a triple-helix structure, thus inhibiting gene transcription. The ASO has potential as described above, but in order to use the ASO in clinical practice, it is required that the stability of the ASO against nucleases be improved and that the ASO be efficiently delivered into a target tissue or cells so as to bind specifically to the nucleotide sequence of a target gene. In addition, the secondary and tertiary structures of genetic mRNA are important factors for specific binding of the ASO, and a region in which formation of the mRNA secondary structure decreases is very advantageous for the ASO to access. Thus, efforts have been made to effectively achieve gene-specific inhibition not only *in vitro* but also *in vivo* by systematically analyzing a region in which formation of the mRNA secondary structure decreases, prior to synthesizing the ASO. These ASOs are more stable than siRNA, a kind of RNA, and have the advantage of being readily soluble in water and physiological saline. To date, three ASOs have been approved by the Federal Drug Administration (FDA) (Jessica, C., J Postdoc Res, 4:35-50, 2016) .

Since the roles of RNA interference (hereinafter referred to as "RNAi") were found, it has been found that RNAi acts on sequence-specific mRNAs in various types of mammalian cells (Barik, S., J Mol. Med. (2005) 83: 764-773). When a long chain of double-stranded RNA is delivered into a cell, the delivered double-stranded RNA is converted into small interfering RNA (hereinafter referred to as "siRNA") processed to 21 to 23 base pairs (bp) by Dicer endonuclease. The siRNA binds to an RNA-induced silencing complex (RISC) and inhibits target gene expression in a sequence-specific manner through a process in which the guide (antisense) strand recognizes and degrades the target mRNA. Technology for inhibiting gene expression using siRNA is used to inhibit target gene expression in target cells and to observe the resulting change, and is effectively used in studies to identify the function of a target gene in target cells. In particular, inhibiting the function of a target gene in infectious viruses or cancer cells may be effectively used to develop a treatment method for the disease of interest. As a result of conducting *in vitro* studies and *in vivo* studies using experimental animals, it has been reported that it is possible to inhibit target gene expression by siRNA.

Bertrand et al. reported that siRNA has a better inhibitory effect on mRNA expression *in vitro* and *in vivo* than an antisense oligonucleotide (ASO) against the same target gene, and that the effect is longer lasting. In addition, regarding the mechanism of action, siRNA regulates target gene expression in a sequence-specific manner by complementary binding to the target mRNA. Thus, siRNA has an advantage over conventional antibody-based drugs or chemical drugs (small-molecule drugs) in that the range of subjects to which the siRNA is applicable can be dramatically expanded (MA Behlke, MOLECULAR THERAPY. 2006 13 (4) : 664-670) .

siRNA has excellent effects and may be used in a wide range of applications, but in order for siRNA to be developed as a therapeutic agent, the *in vivo* stability of siRNA and the cell delivery efficiency thereof should be improved so that siRNA can be effectively delivered to the target cells. In order to improve *in vivo* stability and solve problems associated with non-specific innate immune stimulation of siRNA, studies thereon have been actively attempted by modifying some nucleotides of siRNA or the backbone thereof to have nuclease resistance, or using viral vectors, liposomes, or nanoparticles.

Delivery systems comprising a viral vector such as adenovirus or retrovirus have high transfection efficacy, but have high immunogenicity and oncogenicity. On the other hand, non-viral delivery systems containing nanoparticles have lower cell delivery efficiency than viral delivery systems, but have advantages, including high safety *in vivo,* target-specific delivery, efficient uptake and internalization of RNAi oligonucleotides into cells or tissues, and low cytotoxicity and immune stimulation. Thus, non-viral delivery systems are currently considered a more promising delivery method than viral delivery systems (Akhtar S, J Clin Invest. 2007 December 3; 117(12): 3623-3632).

Among non-viral delivery systems, methods that use nanocarriers are methods in which nanoparticles are formed using various polymers such as liposomes and cationic polymer complexes and in which siRNA is loaded into such nanoparticles (i.e., nanocarriers) and delivered to cells. Among methods that use nanocarriers, frequently used methods include methods that use polymeric nanoparticles, polymer micelles, lipoplexes, and the like. Thereamong, lipoplexes are composed of cationic lipids, and function to interact with the anionic lipids of cellular endosomes to induce destabilization of the endosomes, thus allowing intracellular delivery of the exosomes.

In addition, it is known that the efficiency of siRNA *in vivo* can be increased by conjugating a chemical compound or the like to the end region of the passenger (sense) strand of the siRNA so as to impart improved pharmacokinetic characteristics thereto (J. Soutschek, Nature 11; 432(7014):173-8, 2004). In this case, the stability of the siRNA changes depending on the properties of the chemical compound conjugated to the end of the sense (passenger) or antisense (guide) strand of the siRNA. For example, siRNA conjugated with a polymer compound such as polyethylene glycol (PEG) interacts with the anionic phosphate group of siRNA in the presence of a cationic compound to form a complex, thereby providing a carrier having improved siRNA stability (SH Kim, J Control Release 129(2):107-16, 2008). In particular, micelles composed of a polymer complex have a very small size and a very uniform size distribution compared to other drug delivery systems such as microspheres or nanoparticles, and are spontaneously formed. Thus, these micelles have advantages in that the quality of the micelle formulation is easily managed and reproducibility thereof is easily secured.

In order to improve the intracellular delivery efficiency of siRNA, technology for ensuring the stability of the siRNA and increasing the cell membrane permeability of the siRNA using a siRNA conjugate, obtained by conjugating a hydrophilic compound (e.g., polyethylene glycol (PEG)), which is a biocompatible polymer, to the siRNA via a simple covalent bond or a linker-mediated covalent bond, has been developed (Korean Patent No. 883471). However, even when the siRNA is chemically modified and conjugated to polyethylene glycol (PEG) (PEGylation), it still has low stability *in vivo* and a disadvantage in that it is not easily delivered into a target organ. In order to overcome these disadvantages, a double-stranded oligo RNA structure has been developed, which comprises hydrophilic and hydrophobic compounds bound to an oligonucleotide, particularly double-stranded oligo RNA such as siRNA. This structure forms self-assembled nanoparticles, named SAMiRNA (Self Assembled Micelle Inhibitory RNA), by hydrophobic interaction of the hydrophobic compound (Korean Patent No. 1224828). SAMiRNA technology has advantages over conventional delivery technologies in that homogenous nanoparticles having a very small size may be obtained.

Specifically, in the SAMiRNA technology, PEG (polyethylene glycol) or HEG (hexaethylene glycol) is used as the hydrophilic compound. PEG, a synthetic polymer, is generally used to increase the solubility of medical drugs, particularly proteins, and to regulate the pharmacokinetics of drugs. PEG is a polydisperse material, and a one-batch polymer is made up of different numbers of monomers, and thus exhibits a molecular weight distribution having a Gaussian curve. In addition, the homogeneity of a material is expressed as a polydispersity index (Mw/Mn). In other words, when PEG has a low molecular weight (3 to 5 kDa), it has a polydispersity index of about 1.01, and when PEG has a high molecular weight (20 kDa), it has a high polydispersity index of about 1.2, indicating that the homogeneity of PEG decreases as the molecular weight thereof increases. Thus, when PEG is conjugated to a pharmaceutical drug, there is a disadvantage in that the polydisperse properties of PEG are reflected in the conjugate, and thus it is not easy to verify a single material. Due to this disadvantage, processes for the synthesis and purification of PEG have been improved in order to produce materials having a low polydispersity index. However, when PEG is conjugated to a compound having a low molecular weight, there are problems associated with the polydisperse properties of the compound, including a problem in that it is not easy to confirm whether conjugation was easily achieved (Francesco M.V., DRUG DISCOVERY TODAY(2005) 10(21):1451-1458).

Accordingly, in recent years, SAMiRNA technology (that is, self-assembled nanoparticles) has been improved by forming the hydrophilic compound of the double-stranded RNA structure (constituting SAMiRNA) into basic unit blocks, each comprising 1 to 15 monomers having a uniform molecular weight, and if necessary, a linker, so that a suitable number of the blocks is used according to need. Thus, new types of delivery system technologies, which have small sizes and significantly improved polydisperse properties, compared to conventional SAMiRNA^{™}, have been developed (Korean Patent No. 18162349). It is already known that, when siRNA is injected, the siRNA is rapidly degraded by various enzymes present in the blood, and thus the efficiency of delivery thereof to target cells or tissues is poor. As such, variation in stability and expression inhibition rate depending on target genes also appeared in improved SAMiRNA. Accordingly, in order to more stably and effectively inhibit the expression of a target gene using SAMiRNA, which is composed of improved self-assembled nanoparticles, the present inventors have attempted to enhance the expression inhibitory effect of SAMiRNA on the target gene and the stability of SAMiRNA by applying a double-stranded oligonucleotide comprising the DNA sequence of an ASO as the guide (sense) strand and an RNA sequence as the passenger (antisense sense) sequence.

However, in RNAi drug delivery, conventional RNAi drug delivery methods are mainly based on powder formulations, which have toxicity due to particles themselves and the risk of causing non-specific immune responses and show limited drug delivery efficiency and drug effects, as described above. Accordingly, the present inventors have attempted to develop a drug delivery method particularly suitable for SAMiRNA, which is capable of overcoming these limitations, based on the structural characteristics of SAMiRNA, and have found that, when an ultrasonic nebulizer among various drug delivery means is used, SAMiRNA may be effectively delivered specifically to the lung without cytotoxicity while showing the same concentration, molecular weight, purity, nanoparticle size, and osmolality as those of a stock material (SAMiRNA before nebulization) and maintaining its ability to inhibit a target gene, thereby completing the present invention.

### [Patent Documents]

Korean Patent No. 1224828
Korean Patent No. 1862349

### [Non-Patent Documents]

Mindaugas Rudokas, Med. Princ. Pract. 2016 March; 25:60
Hasan Uludag, Front. Bioeng. Biotechnol. 2020 July; 8:916
Jessica, C., J Postdoc Res. 2016 4:35-50
MA Behlke, MOLECULAR THERAPY. 2006 13(4):664-670
Akhtar S, J Clin Invest. 2007 December 3; 117(12): 3623-3632
SH Kim, J Control Release 129(2):107-16, 2008
Francesco M.V., DRUG DISCOVERY TODAY(2005) 10(21):1451-1458

### Summary of the Invention

An object of the present invention is to provide a pharmaceutical composition for specifically and effectively delivering a double-stranded oligonucleotide structure, which is suitable for the prevention or treatment of respiratory viral infections, viral pulmonary fibrosis, or respiratory disease, to the bronchi and lungs.

To achieve the above object, the present invention provides a pharmaceutical composition for preventing or treating respiratory viral infection, viral pulmonary fibrosis, or respiratory disease comprising a double-stranded oligonucleotide structure comprising a structure represented by the following Structural Formula 1, wherein the pharmaceutical composition is administered using a nebulizer:

[Structural Formula 1] **A-X-R-Y-B**

wherein A represents a hydrophilic compound, B represents a hydrophobic compound, X and Y each independently represent a simple covalent bond or a linker-mediated covalent bond, and R represents a double-stranded oligonucleotide.

### Brief Description of Drawings

FIG. 1 shows the results of comparatively analyzing the molecular weight, purity, nanoparticle size, and osmolarity of SAMiRNA nanoparticles collected from each of an ultrasonic nebulizer and a compressor type nebulizer.
FIG. 2 shows the results of evaluating the cytotoxicity of SAMiRNA nanoparticles collected from an ultrasonic nebulizer.
FIG. 3 shows the results of analyzing the target gene expression inhibitory activity of SAMiRNA nanoparticles collected from an ultrasonic nebulizer.
FIG. 4 shows the results of comparatively analyzing the Cy5 fluorescence, molecular weight, purity, nanoparticle size, and osmolarity of SAMiRNA-Cy5 nanoparticles collected from each of an ultrasonic nebulizer and a compressor type nebulizer.
FIG. 5a shows the results of analyzing the weights of organs, including the lung, spleen, liver and kidney, harvested 24 hours after administering SAMiRNA-Cy5 nanoparticles to hamsters using an ultrasonic nebulizer.
FIG. 5b shows an optical image (left), a fluorescence image (middle) and the results of fluorescence value analysis for organs, including the lung, spleen, liver and kidney, harvested 24 hours after administering SAMiRNA-Cy5 nanoparticles to hamsters using an ultrasonic nebulizer.
FIG. 6 depicts confocal images showing that SAMiRNA nanoparticles were effectively delivered to and distributed in the lung tissue harvested 24 hours after administering SAMiRNA-Cy5 nanoparticles to hamsters using an ultrasonic nebulizer.
FIG. 7 is an experimental scheme showing harvesting of tissue at different time points (1, 24, 48, 96, and 168 hr) after administering SAMiRNA-Cy5 nanoparticles to mice using an ultrasonic nebulizer.
FIG. 7b shows optical images and fluorescence images of the lung, spleen, liver, kidney and heart harvested at different time points (1, 24, 48, 96, and 168 hr) after administering SAMiRNA-Cy5 nanoparticles to mice using an ultrasonic nebulizer.
FIG. 8a shows the results of quantitatively analyzing the fluorescence of the nasal cavity and lung tissues harvested at different time points (1, 24, 48, 96, and 168 hr) after administering SAMiRNA-Cy5 nanoparticles to mice using an ultrasonic nebulizer.
FIG. 8b shows the results of PK analysis of the lung tissues harvested at different time points (1, 24, 48, 96, and 168 hr) after administering SAMiRNA-Cy5 nanoparticles to mice using an ultrasonic nebulizer.
FIG. 9a depicts confocal images showing that SAMiRNA-Cy5 was delivered to and distributed in the lung tissues harvested at different time points (1, 24, 48, 96, and 168 hr) after administering SAMiRNA-Cy5 nanoparticles to mice using an ultrasonic nebulizer.
FIG. 9b depicts confocal images showing that SAMiRNA-Cy5 was delivered to and distributed in the lung tissue harvested 1 hour after administering SAMiRNA-Cy5 nanoparticles to mice using an ultrasonic nebulizer.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

It was found that, among various means for delivering a double-stranded oligonucleotide according to the present invention, an ultrasonic nebulizer is an optimized inhalation drug delivery means that does not affect the physical/chemical properties of the structure. Specifically, as a result of analyzing the physical properties of the double-stranded oligonucleotide structure of the present invention passed through an ultrasonic aerosol inhaler, it was confirmed that the concentration, molecular weight, purity, nanoparticle size, and osmolality were maintained at the same levels as those of a stock material (the structure before passage through the ultrasonic nebulizer). However, it was confirmed that, when a compressor type nebulizer was used, the nanoparticle size, molecular weight, and purity of the structure after passage through the compressor type nebulizer were maintained at the same levels as those of the stock material, but the concentration and osmolality of the structure significantly decreased compared to those of the stock material.

Meanwhile, as a result of evaluating the cytotoxicity of the structure, administered by an ultrasonic nebulizer, by using the human nasal epithelial cell line RPMI2650 and the human lung cancer cell line A549, it could be seen that the structure showed no cytotoxicity even at a high concentration of 50 µM. In addition, in order to evaluate the target gene inhibitory activity of each substance, the RPMI2650 and A549 cells were treated with different concentrations of each substance, followed by analysis, and as a result, it could be confirmed that each substance after passage through an ultrasonic nebulizer exhibited the same target inhibitory activity as that of the substance after passage through the ultrasonic nebulizer.

Finally, in order to examine whether the structure of the present invention is effectively delivered to the lungs *in vivo* by an ultrasonic nebulizer, SAMiRNA was conjugated with Cy5 and administered to hamsters by an ultrasonic nebulizer, and then the lung tissue was harvested and observed. As a result, it was confirmed that the fluorescence of SAMiRNA-Cy5 was found in most cells constituting the lung tissue, and in particular, the strong fluorescence of SAMiRNA-Cy5 was found in the alveoli and bronchi, suggesting that when the SAMiRNA substance was administered by the ultrasonic nebulizer, it was effectively delivered. That is, it was confirmed that the optimized administration of the double-stranded oligonucleotide structure according to the present invention and the nanoparticles self-assembled therefrom could be achieved by the ultrasonic nebulizer.

Therefore, the present invention is directed to a pharmaceutical composition for preventing or treating respiratory viral infection, viral pulmonary fibrosis, or respiratory disease comprising a double-stranded oligonucleotide structure comprising a structure represented by the following Structural Formula 1, wherein the pharmaceutical composition is administered using a nebulizer:

[Structural Formula 1] **A-X-R-Y-B**

wherein A represents a hydrophilic compound, B represents a hydrophobic compound, X and Y each independently represent a simple covalent bond or a linker-mediated covalent bond, and R represents a double-stranded oligonucleotide.

In the present invention, the nebulizer may be an ultrasonic nebulizer.

In the present invention, the double-stranded oligonucleotide may comprise a sense strand and an antisense strand comprising a sequence complementary thereto.

In the present invention, each of the sense strand or the antisense strand may consist of 19 to 31 nucleotides, without being limited thereto.

In the present invention, the sense or antisense strand may be independently DNA or RNA, and may comprise, for example, a sequence in the form of an RNA/RNA, DNA/DNA, or DNA/RNA hybrid.

In the present invention, the sense strand or the antisense strand may comprise a chemical modification,
wherein the chemical modification may be one or more selected from, without limitation to, the group consisting of the following chemical modifications: modification in which an OH group at the 2' carbon position of a sugar structure in one or more nucleotides is substituted with any one selected from the group consisting of a methyl group (-CH₃), a methoxy group (-OCH₃), an amine group (-NH₂), fluorine (-F), a -O-2-methoxyethyl group, an -O-propyl group, an -O-2-methylthioethyl group, an -O-3-aminopropyl group, an -O-3-dimethylaminopropyl group, an -O-N-methylacetamido group, and an -O-dimethylamidooxyethyl group;
modification in which oxygen in a sugar structure in nucleotides is substituted with sulfur;
modification of a bond between nucleotides into any one bond selected from the group consisting of a phosphorothioate bond, a boranophosphate bond and a methyl phosphonate bond; and
modification to PNA (peptide nucleic acid), LNA (locked nucleic acid) or UNA (unlocked nucleic acid).

In the present invention, the chemical modification may contribute to enhancing *in vivo* stability, or conferring nuclease resistance and reducing non-specific immune responses.

In the present invention, one or more phosphate groups, preferably one to three phosphate groups, may be bound to the 5' end of the antisense strand, without being limited thereto.

The double-stranded oligonucleotide according to the present invention is meant to include all substances having general RNAi activity, and it will be obvious to those of ordinary skill in the art that examples of the target gene-specific double-stranded oligonucleotide also include a target gene-specific shRNA. That is, the oligonucleotide may be siRNA, shRNA or miRNA.

The double-stranded oligonucleotide according to the present invention may comprise, at the 3' end of one or both strands, an overhang comprising one or more unpaired nucleotides.

The double-stranded oligonucleotide according to the present invention is preferably in the form of a DNA-RNA hybrid, siRNA (short interfering RNA), shRNA (short hairpin RNA) or miRNA (microRNA), without being limited thereto, and may also include a single-stranded miRNA inhibitor that may act as an antagonist against miRNA.

More preferably, the pharmaceutical composition according to the present invention may comprise a structure represented by the following Structural Formula 2: 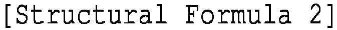 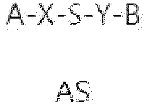 wherein S and AS respectively represent the sense strand and the antisense strand of the double-stranded oligonucleotide, and A, B, X and Y are as defined in Structural Formula 1.

More preferably, the pharmaceutical composition according to the present invention comprise a structure represented by the following Structural Formula 3:

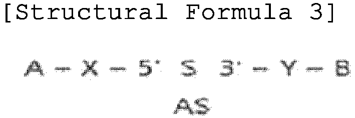

Alternatively, the pharmaceutical composition according to the present invention comprises a structure represented by the following Structural Formula 3':

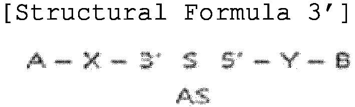

In Structural Formulas 3 and 3' above, A, B, X, Y, S and AS are as defined in Structural Formula 2, and 5' and 3' respectively represent the 5' and 3' ends of the sense strand.

In the present invention, the molecular weight of the hydrophilic compound may be 200 to 10,000, without being limited thereto.

The hydrophilic compound may be selected from the group consisting of polyethylene glycol (PEG), polyvinylpyrrolidone and polyoxazoline, without being limited thereto.

In the present invention, the hydrophilic compound may have a structure represented by the following Structural Formula 4 or Structural Formula 5:

[Structural Formula 4] (A'ₘ-J)ₙ

[Structural Formula 5] (J-A'ₘ)ₙ

wherein A' represents a hydrophilic monomer, J represents a linker that connects m hydrophilic monomers together or connects m hydrophilic monomers with siRNA, m is an integer ranging from 1 to 15, and n is an integer ranging from 1 to 10,
hydrophilic monomer A' is any one compound selected from the following Compounds (1) to (3), and the linker (J) is selected from the group consisting of PO₃⁻, SO₃ and CO_{2:} 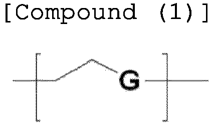
wherein G is selected from the group consisting of CH₂, O, S and NH;

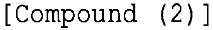

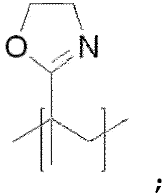

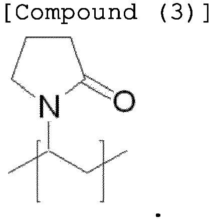

When it has the hydrophilic block represented by Structural Formula 4 or 5 above, the double-stranded oligonucleotide structure according to the present invention may have a structure represented by the following Structural Formula 6 or 7:

[Structural Formula 6] (A'ₘ-J)ₙ-X-R-Y-B

[Structural Formula 7] (J-A'ₘ) ₙ-X-R-Y-B

wherein X, R, Y and B are as defined in Structural Formula 1 above, and A', J, m and n are as defined in Structural Formulas 4 and 5 above.

As the hydrophilic monomer (A') in Structural Formulas 4 and 5 above, one selected from among nonionic hydrophilic polymers may be used without limitation, as long as it is compatible with the purpose of the present invention. Preferably, a monomer selected from among Compound (1) to Compound (3) set forth in Table 1 below may be used, and more preferably, the monomer of Compound (1) may be used. In Compound (1), G is preferably selected from among O, S and NH.

In particular, among hydrophilic monomers, the monomer represented by Compound (1) is very suitable for the production of the structure according to the present invention, because the monomer has advantages in that various functional groups may be introduced to the monomer, and the monomer induces little immune response by having good *in vivo* affinity and excellent biocompatibility, may increase the *in vivo* stability of the double-stranded oligonucleotide comprised in the structure represented by Structural Formula 6 or 7, and may increase the delivery efficiency of the double-stranded oligonucleotide.

**[Table 1] Structure of hydrophilic monomers used in the present invention**

| **Compound (1)** | **Compound (2)** | **Compound (3)** |
|---|---|---|
| 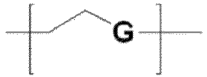 **G is O, S or NH** | 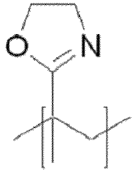 | 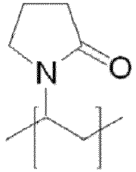 |

The total molecular weight of the hydrophilic compound in Structural Formula 4 to Structural Formula 7 is preferably in the range of 1,000 to 2,000. Thus, for example, when Compound (1) in Structural Formula 6 and Structural Formula 7 is hexaethylene glycol, that is, a compound in which G is O and m is 6, the repeat number (n) is preferably 3 to 5, because the hexaethylene glycol spacer has a molecular weight of 344. Particularly, the present invention is characterized in that a suitable number (represented by n) of repeat units of the hydrophilic group (hydrophilic blocks) represented by (A'ₘ-J) or (J-A'ₘ)ₙ in Structural Formula 4 and Structural Formula 5 may be used as required. The hydrophilic monomer J and linker J comprised in each hydrophilic block may be the same or different between the hydrophilic blocks. The hydrophilic monomer J and linker J comprised in each hydrophilic block may be the same or different between the hydrophilic blocks. In other words, when 3 hydrophilic blocks are used (n = 3), the hydrophilic monomer of Compound (1), the hydrophilic monomer of Compound (2) and the hydrophilic monomer of Compound (3) may be used in the first, second and third blocks, respectively, suggesting that different monomers may be used in all hydrophilic blocks. Alternatively, any one hydrophilic monomer selected from among the hydrophilic monomers of compounds (1) to (3) may also be used in all of the hydrophilic blocks. Similarly, as the linker that mediates the bonding of the hydrophilic monomer, the same linker may be used in the hydrophilic blocks, or different linkers may also be used in the hydrophilic blocks. In addition, m, which is the number of hydrophilic monomers, may also be the same or different between the hydrophilic blocks. In other words, in the first hydrophilic block, three hydrophilic monomers are connected (m=3), and in the second hydrophilic block, five hydrophilic monomers are connected (m=5), and in the third hydrophilic block, four hydrophilic monomers are connected (m=4), suggesting that different numbers of hydrophilic monomers may be used in the hydrophilic blocks. Alternatively, the same number of hydrophilic monomers may also be used in all of the hydrophilic blocks.

In addition, in the present invention, the linker (J) is preferably selected from the group consisting of - PO₃⁻-, - SO₃-, and -CO₂-, without being limited thereto. It will be obvious to those skilled in the art that any linker selected in consideration of the hydrophilic monomer that is used may be used, as long as it is compatible with the purpose of the present invention.

The hydrophobic compound (B) in Structural Formula 3', Structural Formula 6 and Structural Formula 7 functions to form nanoparticles composed of the oligonucleotide structure, through hydrophobic interactions. The hydrophobic compound preferably has a molecular weight of 250 to 1,000, and may be any one selected from the group consisting of a steroid derivative, a glyceride derivative, glycerol ether, polypropylene glycol, a C₁₂-C₅₀ unsaturated or saturated hydrocarbon, diacyl phosphatidylcholine, a fatty acid, a phospholipid, lipopolyamine, a lipid, tocopherol, and tocotrienol, without being limited thereto. It will be obvious to those skilled in the art that any hydrophobic compound may be used, as long as it is compatible with the purpose of the present invention.

The steroid derivative may be selected from the group consisting of cholesterol, cholestanol, cholic acid, cholesteryl formate, cholestanyl formate, and cholesteryl amine, and the glyceride derivative may be selected from among mono-, di-, and tri-glycerides and the like. Here, the fatty acid of the glyceride is preferably a C₁₂-C₅₀ unsaturated or saturated fatty acid.

In particular, among the hydrophobic compounds, a saturated or unsaturated hydrocarbon or cholesterol is preferably used because it may be easily bound in a step of synthesizing the double-stranded oligonucleotide structure according to the present invention. Most preferably, a C₂₄ hydrocarbon, particularly a hydrophobic hydrocarbon containing a disulfide bond, is used.

The hydrophobic compound may be bound to the distal end of the hydrophilic compound, and may be bound to any position on the sense or antisense strand of the double-stranded oligonucleotide.

The hydrophilic or hydrophobic compound in Structural Formulas 1 to 3', 6 and 7 according to the present invention is bound to the double-stranded oligonucleotide by a single covalent bond or a linker-mediated covalent bond (X or Y). The linker that mediates the covalent bond is covalently bound to the hydrophilic or hydrophobic compound at the end of the double-stranded oligonucleotide, and is not specifically limited, as long as it provides a degradable bond in a specific environment if required. Therefore, the linker that is used in the present invention may be any compound that is bound in order to activate the double-stranded oligonucleotide and/or the hydrophilic (or hydrophobic) compound in the process of producing the double-stranded oligonucleotide structure according to the present invention. The covalent bond may be either one of a non-degradable bond and a degradable bond. Here, examples of the non-degradable bond include, but are not limited to, an amide bond and a phosphate bond, and examples of the degradable bond include, but are not limited to, a disulfide bond, an acid-degradable bond, an ester bond, an anhydride bond, a biodegradable bond, and an enzyme-degradable bond.

In addition, in the structure comprising the double-stranded oligonucleotide according to the present invention, an amine or polyhistidine group may additionally be introduced to the distal end of the hydrophilic compound bound to the oligonucleotide in the structure.

This facilitates intracellular uptake and endosomal escape of a carrier comprising the structure comprising the double-stranded oligonucleotide according to the present invention, and it has already been reported that the introduction of an amine group and a polyhistidine group may be used to facilitate the intracellular uptake and endosomal escape of carriers such as quantum dots, dendrimers or liposomes.

Specifically, it is known that a primary amine group introduced to the end or outside of a carrier is protonated at biological pH while forming a conjugate by interaction with a negatively charged gene, and that endosomal escape is facilitated due to an internal tertiary amine having a buffering effect at low pH after intracellular uptake, whereby the carrier can be protected from lysosomal degradation (Gene Delivery and Expression Inhibition Using Polymer-Based Hybrid Material, Polymer Sci. Technol., Vol. 23, No. 3, pp 254-259) .

In addition, it is known that histidine, a non-essential amino acid, has an imidazole ring (pKa = 6.04) at the residue (-R) thereof, and thus has an effect of increasing buffering capacity in endosomes and lysosomes, and thus histidine modification may be used in non-viral gene carriers, including liposomes, in order to increase endosomal escape efficiency (Novel histidine-conjugated galactosylated cationic liposomes for efficient hepatocyte selective gene transfer in human hepatoma HepG2 cells. J. Controlled Release 118, pp. 262-270).

The amine group or polyhistidine group may be connected to the hydrophilic compound or the hydrophilic block by one or more linkers.

When the amine group or polyhistidine group is introduced to the hydrophilic compound of the double-stranded oligonucleotide structure represented by Structural Formula 1 according to the present invention, the structure may have a structure represented by the following Structural Formula 8:

[Structural Formula 8] P-J₁-J₂-A-X-R-Y-B

wherein A, B, R, X and Y are as defined in Structural Formula 1 above,
P represents an amine group or a polyhistidine group, and J₁ and J₂ are linkers, each of which may be independently selected from among a simple covalent bond, PO₃⁻, SO₃, CO₂, a C₂₋₁₂ alkyl, alkenyl and alkynyl, without being limited thereto. It will be obvious to those skilled in the art that any linkers selected in consideration of the hydrophilic compound used herein may be used as J₁ and J₂, as long as they are compatible with the purpose of the present invention.

Preferably, when an amine group is introduced, J₂ is preferably a simple covalent bond or PO₃⁻, and J₁ is preferably a C₆ alkyl, without being limited thereto

In addition, when a polyhistidine group is introduced, it is preferred that J₂ in Structural Formula 8 be a simple covalent bond or PO₃⁻, and that J₁ be Compound (4), without being limited thereto.

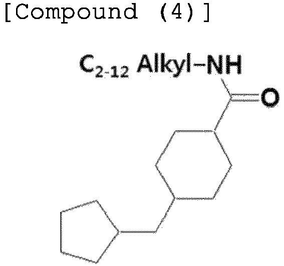

In addition, when the hydrophilic compound of the double-stranded oligonucleotide structure represented by Structural Formula 8 is the hydrophilic block represented by Structural Formula 5 or 6 and an amine group or a polyhistidine group is introduced thereinto, the double-stranded oligonucleotide structure may have a structure represented by the following Structural Formula 9 or 10:

[Structural Formula 9] P-J₁-J₂- (A'ₘ-J)ₙ -X-R-Y-B

[Structural Formula 10] P-J₁-J₂- (J-A'ₘ)ₙ -X-R-Y-B

wherein X, R, Y, B, A', J, m and n are as defined in Structural Formula 4 or 5 above, and P, J₁ and J₂ are as defined in Structural Formula 8 above.

In particular, the hydrophilic compound in Structural Formula 9 and Structural Formula 10 is preferably bound to the 3' end of the sense strand of the double-stranded oligonucleotide. In this case, Structural Formula 8 to Structural Formula 10 may correspond to the following Structural Formula 11 to Structural Formula 13:

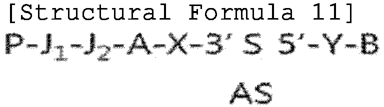

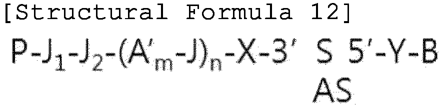

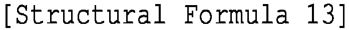

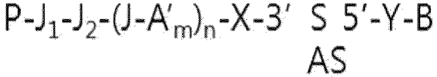

wherein X, R, Y, B, A, A' J, m, n, P, J₁ and J₂ are as defined in Structural Formula 8 to Structural Formula 10 above, and 5' and 3' respectively represent the 5' end and the 3' end of the sense strand of the target gene-specific double-stranded oligonucleotide.

An amine group that may be introduced in the present invention may be a primary, secondary or tertiary amine group. In particular, a primary amine group is preferably used. The introduced amine group may be present as an amine salt. For example, a salt of the primary amine group may be present as NH₃⁺.

In addition, a polyhistidine group that may be introduced in the present invention preferably comprises 3 to 10 histidines, more preferably 5 to 8 histidines, and most preferably 6 histidines. In addition to histidine, one or more cysteines may be included.

Meanwhile, when a targeting moiety is provided in the double-stranded oligonucleotide structure comprising the target gene-specific oligonucleotide according to the present invention and nanoparticles formed therefrom, it may promote the efficient delivery of the structure or nanoparticles to target cells, so that the structure or nanoparticles may be delivered to the target cells even at a relatively low concentration, thus exhibiting a strong effect of regulating target gene expression.

Accordingly, the present invention provides a double-stranded oligo RNA structure in which a ligand (L), particularly a ligand having the property of binding specifically to a receptor that enhances target cell internalization by receptor-mediated endocytosis (RME), is further bound to the structure represented by any one of Structural Formulas 1 to 3', 6 and 7. For example, a structure in which a ligand is bound to the double-stranded oligo RNA structure represented by Structural Formula 1 has a structure represented by the following Structural Formula 14:

[Structural Formula 14] (Lᵢ-Z)-A-X-R-Y-B

wherein A, B, X and Y are as defined in Structural Formula 1 above, L is a ligand having the property of binding specifically to a receptor that enhances target cell internalization by receptor-mediated endocytosis (RME), and "i" is an integer ranging from 1 to 5, preferably from 1 to 3.

The ligand in Structural Formula 14 may preferably be selected from among: target receptor-specific antibodies, aptamers and peptides, which have the RME property of enhancing target cell internalization; folate (the term "folate" is generally used interchangeably with folic acid, and the term "folate" as used herein means folate that is in a natural form or is activated in the human body); and chemical compounds, including hexosamines such as N-acetyl galactosamine (NAG), and sugars or carbohydrates such as glucose and mannose, without being limited thereto.

In addition, the hydrophilic compound A in Structural Formula 14 above may be used in the form of the hydrophilic block represented by Structural Formula 4 or 5.

In the present invention, the double-stranded oligonucleotides represented by R (or S and AS) in Structural Formulas 1 to 3', Structural Formula 6 and Structural Formula 7 may be any oligonucleotide that specifically inhibits the expression of a gene selected from the group consisting of amphiregulin, RelA/p65, and SARS-CoV-2, without being limited thereto. Specifically, the double-stranded oligonucleotide represented by R (or S and AS) may specifically inhibit the expression of amphiregulin gene, and may comprise an amphiregulin-targeting sequence. The amphiregulin-targeting sequence may comprise, for example, the sequence of SEQ ID NO(s): 5 and/or 6. Specifically, it may comprise the sense sequence of SEQ ID NO: 5 and the antisense sequence of SEQ ID NO: 6.

The double-stranded oligonucleotide represented by R (or S and AS) may comprise a sequence targeting SARS-CoV-2. The sequence targeting SARS-CoV-2 may comprise, for example, a sequence selected from the group consisting of SEQ ID NOs: 11 to 30.

The present invention is suitable for the prevention or treatment of various respiratory diseases, and is particularly suitable as a method of administering a composition for preventing or treating respiratory viral infection, pulmonary fibrosis caused by viral infection, or respiratory disease.

In the present invention, the respiratory virus may be COVID-19, without being limited thereto. In the present invention, the pulmonary fibrosis caused by viral infection is an example of the sequelae caused by viral infection, and the present invention may be used to administer a composition for preventing or treating sequelae caused by viral infection, particularly respiratory viral infection, in addition to pulmonary fibrosis.

In the present invention, the respiratory disease may be selected from the group consisting of interstitial lung disease, chronic obstructive pulmonary disease (COPD), pneumonia, asthma, acute and chronic bronchitis, allergic rhinitis, bronchitis, bronchiolitis, pharyngitis, tonsillitis, and laryngitis, without being limited thereto.

In the present invention, the double-stranded oligonucleotide structure may form self-assembled nanoparticles, which are 10 to 100 nm in size and have a neutral charge, in an aqueous solution for administration.

In the present invention, the nanoparticle may be composed of a mixture of double-stranded oligonucleotide structures comprising double-stranded oligonucleotides comprising different sequences.

For administration, the composition of the present invention may further comprise one or more pharmaceutically acceptable carriers, in addition to the above-described active ingredient. The pharmaceutically acceptable carriers should be compatible with the active ingredient, and may be selected from among physiological saline, sterile water, Ringer's solution, buffered saline, dextrose solution, maltodextrin solution, glycerol, ethanol, and a mixture of two or more thereof. If necessary, the composition may comprise other conventional additives such as an antioxidant, a buffer or a bacteriostatic agent. In addition, a diluent, a dispersing agent, a surfactant, a binder and a lubricant may additionally be added to the composition to prepare injectable formulations such as an aqueous solution, a suspension, and an emulsion. In particular, the composition is preferably provided as a lyophilized formulation. For the preparation of a lyophilized formulation, a conventional method known in the art to which the present invention pertains may be used, and a stabilizer for lyophilization may also be added. Furthermore, the composition may preferably be formulated depending on each disease or component by a suitable method known in the art or by a method disclosed in Remington's Pharmaceutical Science, Mack Publishing Company, Easton PA.

The pharmaceutical composition of the present invention is preferably administered parenterally, particularly preferably administered to the lungs by inhalation into the bronchi. The dose of the composition according to the present invention may vary depending on the patient's weight, age, sex, health condition and diet, the duration of administration, the mode of administration, excretion rate, severity of disease, or the like, and may be easily determined by those skilled in the art.

### Examples

Hereinafter, the present invention will be described in more detail with reference to examples. These examples are only for explaining the present invention in more detail, and it will be obvious to those of ordinary skill in the art that the scope of the present invention is not limited by these examples. Accordingly, the substantial scope of the present invention will be defined by the appended claims and equivalents thereto.

### Example 1. Synthesis of double-stranded oligonucleotide structure

A double-stranded oligonucleotide structure (SAMiRNA) produced in the present invention has a structure represented by the following structural formula: 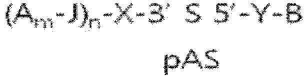 C₂₄-5' S 3'-(hexaethyleneglycol-PO₃⁻)₃-hexaethyleneglycol AS 5'-PO₄

For synthesis of the sense strain of a monoSAMiRNA double-stranded oligo structure, 3,4,6-triacetyl-1-hexa(ethylene glycol)-CPG was used as a support, and three demethoxytrityl (DMT) hexaethylene glycol phosphoramidates as hydrophilic monomers were continuously bound to the support through a reaction. Next, synthesis of DNA was performed, and then hydrophobic C₂₄ (C₁₉-S-S-C₆) containing a disulfide bond was bound to the 5' end region, thereby synthesizing a sense strand in which hexaethyleneglycol-(-PO₃⁻hexaethyleneglycol)₃ is bound to the 3' end and C₂₄ (C₁₈-S-S-C₆) is bound to the 5' end.

After completion of the synthesis, the synthesized DNA single strand and DNA-polymer structure were detached from the CPG by treatment with 28%(v/v) ammonia in a water bath at 60°C, and then protective residues were removed by a deprotection reaction. The DNA single strand, the DNA-polymer structure and the ligand-bound DNA-polymer structure were separated from the reaction products by high-performance liquid chromatography (HPLC), and the molecular weights thereof were measured using a MALDI-TOF mass spectrophotometer (MALDI TOF-MS, SHIMADZU, Japan) to confirm whether they matched the nucleotide sequence and DNA-polymer structure desired to be synthesized.

For synthesis of the antisense strand, synthesis of RNA having a sequence complementary to the sense strand was performed using a linker (UnyLinker^{™})-bound support, and then an antisense strand having a phosphate group (PO₄) bound to the 5' end region was synthesized.

After completion of the synthesis, the synthesized RNA single strand and RNA-polymer structure were detached from the CPG by treatment with 28%(v/v) ammonia in a water bath at 60°C, and then protective residues were removed by a deprotection reaction. After removal of the protective residues, the RNA single strand and the RNA-polymer structure were treated with N-methylpyrrolidone, trimethylamine and triethylamine trihydrofluoride at a volume ratio of 10:3:4 in an oven at 70°C to remove 2'-TBDMS (tert-butyldimethylsilyl). The RNA single strand, the RNA-polymer structure and the ligand-bound RNA-polymer structure were separated from the reaction products by high-performance liquid chromatography (HPLC), and the molecular weights thereof were measured using a MALDI-TOF mass spectrophotometer (MALDI TOF-MS, SHIMADZU, Japan) to confirm whether they matched the nucleotide sequence and RNA-polymer structure desired to be synthesized. Thereafter, to produce each double-stranded oligo structure, the sense strand and the antisense strand were mixed together in equal amounts, added to 1X PBS (phosphate buffer saline, 30 mg/1ml (1x)PBS) annealing buffer (30 mM HEPES, 100 mM potassium acetate, 2 mM magnesium acetate, pH 7.0 to 7.5), allowed to react in a water bath at 90°C for 5 minutes, and cooled slowly to room temperature, thereby producing the desired SAMiRNA. Annealing of the produced double-stranded oligo RNA structures was confirmed by high-performance liquid chromatography (HPLC, Non-Denaturation). Information on the sequence of the SAMiRNA-AREG synthesized for efficacy evaluation is shown in Table 2 below.

**[Table 2] Human amphiregulin (AREG)-targeting sequence**

| | |
|---|---|
| hAREG sense (DNA) | 5'- CTGGGAAGCGTGAACCATT -3' (SEQ ID NO: 5) |
| hAREG antisense (RNA) | 5'- AAUGGUUCACGCUUCCCAG -3' (SEQ ID NO: 6) |

### Example 2. Collection of SAMiRNA nanoparticles from nebulizer

In the present invention, SAMiRNA was collected from each of an ultrasonic nebulizer and a compressor type nebulizer.

1 ml of SAMiRNA was placed in the drug chamber of the ultrasonic nebulizer (Meshnet2, China), and a mouthpiece was connected to the chamber. The aerosol inhaler was operated in a state in which the tip of the mouthpiece was placed in a test tube for collection. To collect the nebulized SAMiRNA, ice was put in an icebox and placed around the test tube, and the nebulizer was operated for 10 minutes, and collection of the nebulized SAMiRNA was carried out for a total of 20 minutes in consideration of the liquefaction time of the nebulized SAMiRNA.

1 ml of SAMiRNA was placed in the drug chamber of a compressor type nebulizer (Philips non-heated nebulizer, US), a rubber hose was connected to the chamber, and a test tube for collection was coupled to the end of the rubber hose. To collect the nebulized SAMiRNA, ice was put in an icebox and placed around the test tube, and the nebulizer was operated for 10 minutes, and collection of the nebulized SAMiRNA was carried out for a total of 20 minutes in consideration of the liquefaction time of the nebulized SAMiRNA.

### Example 3. Analysis of physical properties of SAMiRNA nanoparticles collected from each nebulizer

The RNA single-strand, the RNA-polymer structure and the ligand-bound RNA-polymer structure were separated from each collected SAMiRNA by high-performance liquid chromatography, and analyzed using a MALDI-TOF mass spectrometer to confirm whether the intended sequence was present. The osmolarity of the collected SAMiRNA was measured with Osmomat 3000 (Gonotec, Germany) to confirm whether the osmolality of the captured SAMiRNA was the same as that of physiological saline, which does not cause a change in osmotic pressure even when it enters the blood vessel directly. The particle size of the collected SAMiRNA was analyzed using a Zetasizer NANO-ZS (Malvern, UK), which is a dynamic light scattering (DLS) device, and The qNano Gold (IZON, New Zealand) using tunable resistive pulse detection (TRPS) according to the manufacturer's protocols.

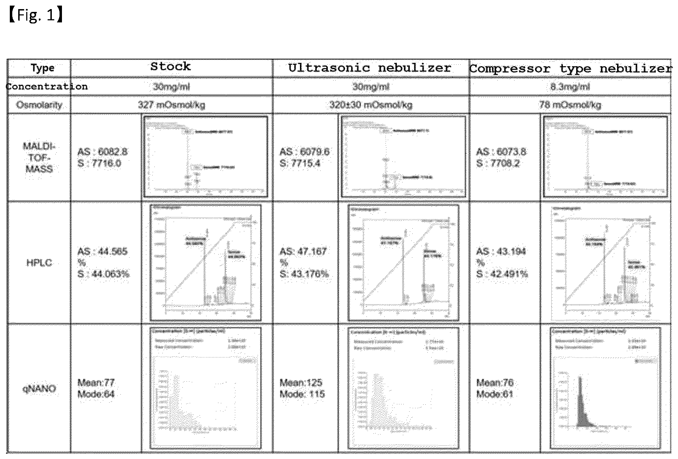

As a result, it could be confirmed that the concentration, molecular weight, purity, nanoparticle size, and osmolality of the SAMiRNA nanoparticles collected from the ultrasonic nebulizer were the same as those of the SAMiRNA before passage through the ultrasonic nebulizer, whereas, in the case of the SAMiRNA collected from the compressor-type nebulizer, the nanoparticle size, molecular weight, and purity of the SAMiRNA were the same as those of the SAMiRNA before passage through the compressor type nebulizer, but the concentration decreased by 3.6 times (8.3 mg/ml), and the osmolality was also significantly lower than that of physiological saline (FIG. 1).

### Example 4. Evaluation of cytotoxicity of SAMiRNA nanoparticles collected from each nebulizer

Human lung cancer cell line A549 (CCL-185, ATCC, US) and human nasal epithelial cell line RPMI2650 (Korea Cell Line Bank, KR) were used to evaluate the cytotoxicity of the SAMiRNA nanoparticles collected from each nebulizer. A549 cells were cultured using F12K medium (Gibco, US) containing 10% fetal bovine serum (Hyclone, US) and 1% penicillin-streptomycin (Hyclone, US) at 37°C under 5% CO₂. RPMI2650 cells were cultured in RPMI1640 medium (Hyclone, US) containing 10% fetal bovine serum (Hyclone, US) and 1% penicillin-streptomycin (Hyclone, US) at 37°C under 5% CO₂. The A549 cells were seeded in a 96-well plate (Falcon, US) at a density of 3 × 10³ cells/well, and the RPMI2650 cells were seeded in a 96-well plate (Falcon, US) at a density of 1 × 10⁴ cells/well. The next day, the cells were treated with different concentrations (0, 1, 5, 10, 20, and 50 µM) of the SAMiRNA collected from each nebulizer. After incubation for 96 hours, experimental analysis was performed using a WST assay kit (DOGEN, KR) according to the manufacturer's protocol in order to measure the cytotoxicity of the SAMiRNA.

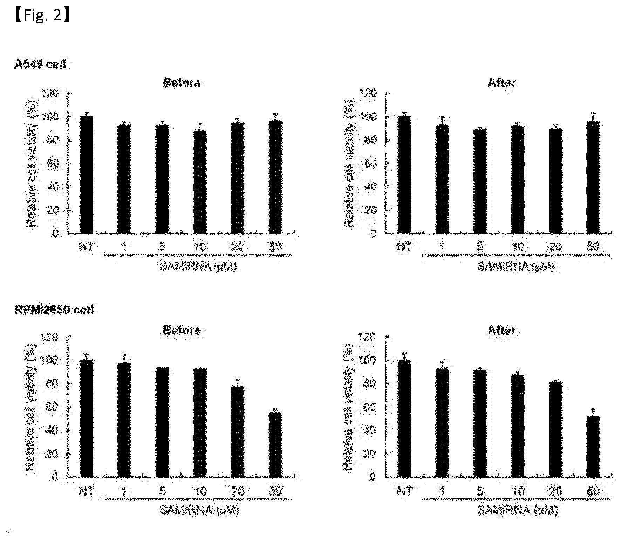

As a result, it was confirmed that cytotoxicity was not found in the two types of cell lines before and after passage through the ultrasonic nebulizer up to a concentration of 20 µM, and in the case of RPMI2650 cells, some cytotoxicity was found only at a concentration of 50 µM (FIG. 2).

### Example 5. Evaluation of target gene expression inhibitory activity of SAMiRNA nanoparticles collected from each ultrasonic nebulizer

### 5-1. Treatment of cells with SAMiRNA nanoparticles collected from ultrasonic nebulizer

A549 cells (CCL-185, ATCC, US) and RPMI2650 cells (Korea Cell Line Bank, KR) were used to analyze whether the SAMiRNA nanoparticles collected from the ultrasonic nebulizer effectively inhibited target gene expression. A549 cells were cultured using F12K medium (Gibco, US) containing 10% fetal bovine serum (Hyclone, US) and 1% penicillin-streptomycin (Hyclone, US) at 37°C under 5% CO₂. RPMI2650 cells were cultured RPMI1640 medium (Hyclone, US) containing 10% fetal bovine serum (Hyclone, US) and 1% penicillin-streptomycin (Hyclone, US) at 37°C under 5% CO₂. The A549 cells were seeded in a 12-well plate (Falcon, US) at a density of 5 × 10⁴ cells/well, and the RPMI2650 cells were seeded in a 12-well plate (Falcon, US) at a density of 1.2 × 10⁵ cells/well. The next day, the cells were treated with different concentrations (0, 0.1, 0.5, 1, 5, and 10 µM) of the SAMiRNA collected from the ultrasonic nebulizer.

### 5-2. Analysis of target gene expression treatment with SAMiRNA nanoparticles collected from ultrasonic nebulizer

According to the method described in Example 5-1, each cell line was treated with the SAMiRNA nanoparticles collected from the ultrasonic nebulizer. After 24 hours of incubation, total RNA was extracted from the cell lysate using a universal RNA extraction kit (Bioneer, KR). Using this RNA as a template, the mRNA expression levels of human AREG and RPL13A (human reference qPCR primer set, Bioneer, KR) were analyzed by qRT-PCR using *AccuPower* GreenStar^{™} RT-qPCR Master Mix (Bioneer, KR) according to the manufacturer's protocol. Based on the Ct values of the two genes obtained after qPCR array, the relative amount (fold change) of AREG mRNA in the test group compared to that in the control group was analyzed by the 2(-Delta Delta C(T)) method [Livak KJ, Schmittgen TD. 2001. Analysis of relative gene expression data using real-time quantitative PCR and the 2(-Delta Delta C(T)) Method. Methods. Dec; 25(4):4 02-8]. The primer sequences for each gene are as follows (Table 3).

**[Table 3] Primer sequences for human AREG and human RPL13A (internal control)**

| | |
|---|---|
| hAREG-forward | 5'- ACACCTACTCTGGGAAGCGT -3' (SEQ ID NO: 1) |
| hAREG-reverse | 5'- GCCAGGTATTTGTGGTTCGT -3' (SEQ ID NO: 2) |
| hRPL13A-forward | 5'- CCAGCAATCAAGTTTGCCTA -3' (SEQ ID NO: 3) |
| hRPL13A-reverse | 5'- GTGGTGGTGGTGGTAATTCA -3' (SEQ ID NO: 4) |

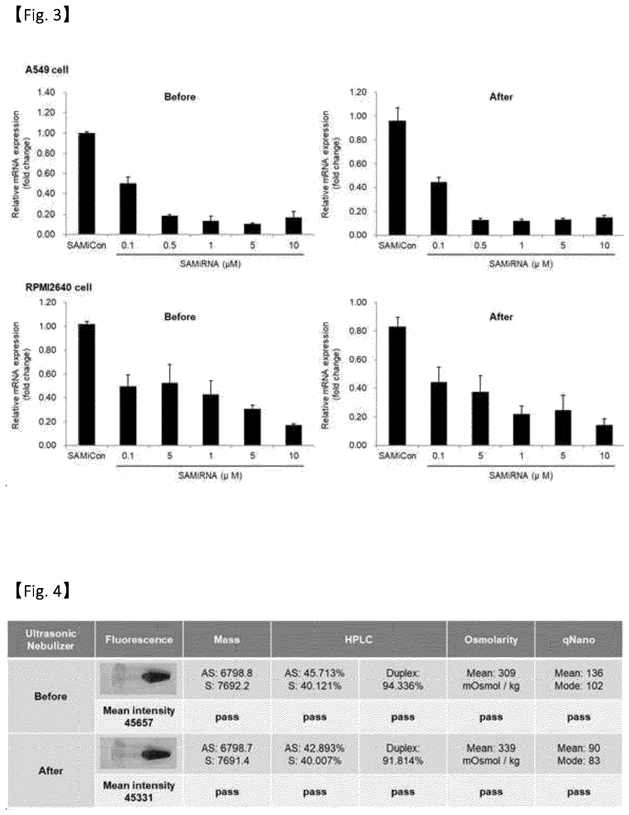

As a result, it was observed that both the SAMiRNA nanoparticles before and after passage through the ultrasonic nebulizer showed AREG mRNA inhibitory efficacy in the two cell lines in a concentration-dependent manner (FIG. 3). In summary, it was confirmed that the SAMiRNA nanoparticles collected from the ultrasonic nebulizer maintained their physical and chemical properties, did not show cytotoxicity, and maintained the same target gene inhibitory activity as that before passage through the ultrasonic nebulizer.

### Example 6. Evaluation of delivery efficacy of SAMiRNA nanoparticles administered using an ultrasonic nebulizer in animal models

### 6-1. Analysis of properties of fluorescently labeled SAMiRNA (SAMiRNA-Cy5) nanoparticles collected after passage through ultrasonic nebulizer

SAMiRNA-Cy5 nanoparticles collected from the ultrasonic nebulizer according to the method of Example 2 were analyzed according to the method of Example 3. As a result, it was confirmed that the SAMiRNA-Cy5 nanoparticles collected from the ultrasonic nebulizer had the same fluorescence value, concentration, molecular weight, purity, nanoparticle size, and osmolality as those of the material before passage through the aerosol nebulizer (FIG. 4).

### 6-2. Evaluation of delivery efficacy of SAMiRNA-Cy5 nanoparticles administered using ultrasonic nebulizer in hamster animal model

Hamsters were used as laboratory animals. Hamsters (5 weeks old, male) were purchased from Central Laboratory Animal Inc., acclimatized for 1 week, and used in the experiments. After the mouthpiece of the ultrasonic nebulizer was attached to the hamster's face, SAMiRNA-Cy5 (5 mg/ml) or 1 ml of PBS was placed in the drug chamber, and the ultrasonic nebulizer was operated for 2 minutes and 30 seconds while the hamster was exposed to the nebulizer. 24 hours after exposure to the ultrasonic nebulizer, the lung, liver, spleen, and kidney were harvested and weighed. Fluorescence imaging and fluorescence value analysis of each organ were performed using the Davinch-Invivo^{™} imaging system (Davinch-K, KR).

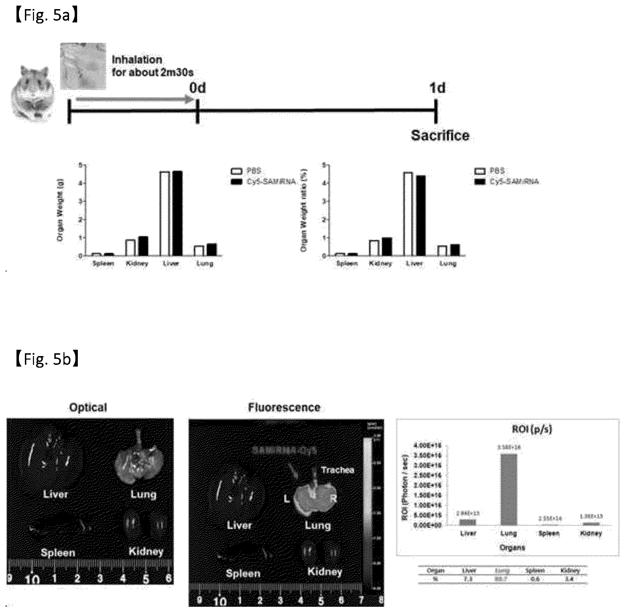

As a result, it was observed that the weights and weight ratios of the lung, spleen, liver and kidney harvested from the hamster to SAMiRNA-Cy5 by the ultrasonic nebulizer did not change compared to those of the organs of the hamster exposed to PBS by the ultrasonic nebulizer (FIG. 5a). In addition, fluorescence images of the lung, spleen, liver, and kidney tissues harvested from the hamster exposed to SAMiRNA-Cy5 by the ultrasonic nebulizer were analyzed using the Davinch-Invivo^{™} system, and as a result, it was confirmed that almost no fluorescence was found in the liver, spleen and kidney, but strong fluorescence was found in the lung (FIG. 5b), indicating that the SAMiRNA nanoparticles were effectively delivered to the lung through the airways by the ultrasonic nebulizer.

### 6-3. Analysis of lung tissue biodistribution of SAMiRNA nanoparticles administered by ultrasonic nebulizer in hamster animal model

Immunofluorescence staining was performed on the lung tissue harvested from the hamsters exposed to each of PBS and SAMiRNA-Cy5 in Example 6-2 by the ultrasonic nebulizer. The tissue was stored for one day in 10% neutral buffered formalin (Sigma, US) for fixation, and then dehydrated sequentially in 10%, 20%, and 30% sucrose (Sigma, US) solutions. Each lung tissue sample was placed in a base mold (Thermo Scientific, US) containing OCT compound (Sakura Finetek, US), and a stainless plate was placed in a container containing liquid nitrogen, and the base mold was placed thereon to completely freeze the OCT compound. The frozen tissue was stored at -70°C and placed at -20°C for 30 min before microtome sectioning to facilitate tissue sectioning. The tissue sections with a thickness of 14 um were placed on a slide and dried for 1 hour. Next, the tissue was incubated for 10 minutes with 0.1% Triton-X100 (Sigma, US) solution for cell permeabilization, blocked with a solution containing 5% normal goat serum (Abcam, GB) and 1% BSA (Sigma, US) for 1 hour, and incubated with alpha-actin-2 antibody (Sigma, US) at 4°C for one day. After washing with PBS, the tissue was incubated with the secondary antibody anti-mouse-Alexa Fluor 488 (Invitrogen, US) for 1 hour, followed by washing. The tissue was incubated with 1 µM DAPI (Sigma, US) solution for 10 minutes and washed, and a mounting solution (Thermo Scientific, US) was dropped thereon and covered with a cover glass (VWR, US). For fluorescence analysis of the stained tissue, the stained tissue was analyzed using spinning disk confocal microscopy (Dragonfly high-speed confocal image platform, Andor, GB).

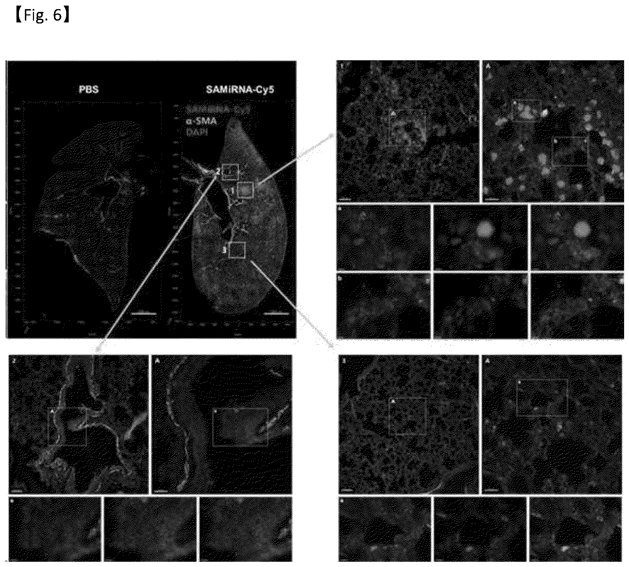

As a result, fluorescence (SAMiRNA-Cy5) was found throughout the lung of the hamster exposed to SAMiRNA-Cy5 by the ultrasonic nebulizer. As a result of observing each part at high magnification, it was confirmed that SAMiRNA-Cy5 was well delivered to most of the cells constituting the lung, and in particular, it was confirmed that strong fluorescence was found in the alveoli and bronchi. (FIG. 6). It was confirmed that, when the SAMiRNA nanoparticles were administered using the ultrasonic nebulizer, they were effectively delivered to the lung, suggesting that the double-stranded oligonucleotide structure and nanoparticles are an optimized siRNA platform for the ultrasonic nebulizer.

### 6-4. Time-dependent fluorescence image analysis of tissues after administration of SAMiRNA-Cy5 nanoparticles by ultrasonic nebulizer in mouse animal model

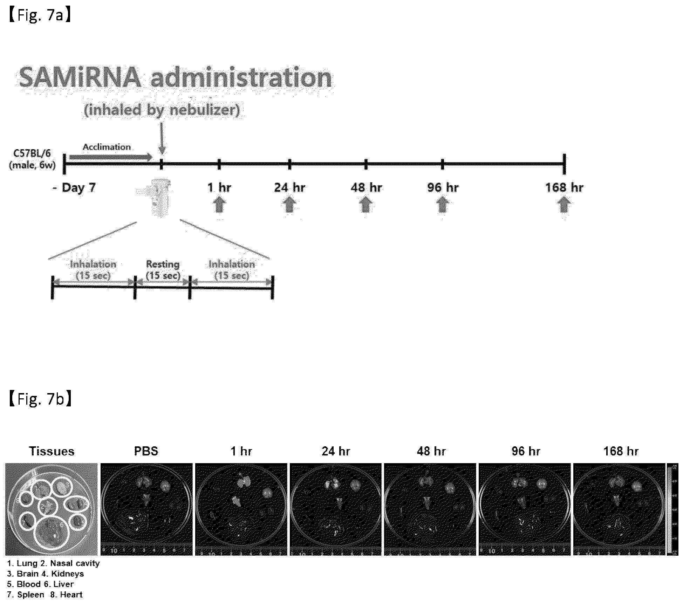

C57BL/6 mice were used as laboratory animals. Mice (6 weeks old, male) were purchased from Daehan Biolink and acclimatized for 1 week before the experiment. As the fluorescently labeled SAMiRNA-Cy5, the material used in Example 6-1 was used. After the mouthpiece of the ultrasonic nebulizer was attached to the face of each mouse, SAMiRNA-Cy5 (2 mg) or PBS was placed in the drug chamber and administered by operating the ultrasonic nebulizer for 30 seconds (15 seconds inhalation - 15 seconds resting - 15 seconds inhalation) (FIG. 7a). After administration, the nasal cavity, lung, liver, spleen, kidney and heart were harvested at different time points (0, 1, 24, 48, 96, and 168 hours) and weighed, and then fluorescence image analysis was performed using the Davinch-Invivo^{™} imaging system (Davinch-K, KR). Fluorescence image analysis was performed on all tissues (harvested at different time points) under the same conditions (fluorescence intensity, exposure time, etc.).

As a result, it was confirmed that the strongest fluorescence was found in the mouse nasal cavity and lung tissues harvested 1 hour after administration by the ultrasonic nebulizer, indicating that the SAMiRNA was effectively delivered to the nasal cavity and lung tissues. On the other hand, it was confirmed that no fluorescence signal was found in the liver, kidney, heart, spleen, brain, blood, etc. It was observed that, after 24 hours, the fluorescence intensity of the nasal cavity and lung tissues was significantly lower than that at 1 hour, and after 48 hours, the fluorescence intensity of the nasal cavity and lung tissues started to gradually decrease, and after 168 hours, almost no fluorescence was found even in the lung tissues (FIG. 7b).

### 6-5. Quantitative analysis of SAMiRNA nanoparticles administered by ultrasonic nebulizer in nasal cavity and lung tissues of mouse animal model

Fluorescence quantitative analysis and PK analysis were performed using the nasal cavity and lung tissues harvested from the mice exposed to each of PBS and SAMiRNA-Cy5 by the ultrasonic nebulizer in Example 6-4. Each harvested tissue was measured, and the whole tissue was placed in a round bottom tube (SPL, KR). 1 ml of tissue lysis buffer (Bioneer, KR) was added to the tissue which was then homogenized using a homogenizer (IKA, DE). After 15 minutes of incubation on ice, the tissue was centrifuged at 14,000 rpm at 4°C for 15 minutes, and then the supernatant was transferred to and stored in a 1.5-ml amber micro-centrifuge tube (Axigen, US). Standard samples for fluorescence quantitative analysis were prepared by spiking SAMiRNA-Cy5 into the PBS-treated tissue lysate at different concentrations (0.1, 0.5, 0.25, and 0.125 pg/ml). 100 µl of each of the standard and the sample was dispensed into each well of black 96-well microplates (Corning Costar, US), and then the fluorescence intensity (excitation wavelength 645 nm, emission wavelength 675 nm) thereof was measured with a microplate reader (TECAN, CH) and substituted into the determined standard curve, thereby determining the amount of SAMiRNA-Cy5 remaining in each tissue. For PK analysis of SAMiRNA, the lysed tissue samples were homogenized once more using QIAshredder (QIAGEN). Ago2 immunoprecipitation was performed from the tissue lysate using the MagListo^{™} Protein G Kit (Bioneer, KR) conjugated with an anti-mouse AGO2 antibody (Sigma, US). The obtained RISC-loaded siRNA was subjected to absolute quantitative analysis by stem-loop RT-qPCR. Conversion into cDNA was performed using Taqman MicroRNA Reverse transcription kit (Applied Biosystem, US), Mygenie^{™} 96 (Bioneer, KR) and SAMiRNA-mRelA antisense RT primer (Bioneer, KR), and then qPCR was performed using AccuPower^{®} Plus DualStar^{™} qPCR Master Mix (Bioneer, KR), 600 nM qPCR primer (Bioneer, KR), 300 nM probe, and Exicycler^{™} 96 (Bioneer, KR). The standard used for quantitation was spiked into the untreated sample, and then used analysis in the same manner. The RT and qPCR primer sequences used in analysis are as follows (Table 4) .

**[Table 4] RT and qPCR primer sequences for mouse RELA**

| | |
|---|---|
| mRELA RT primer | 5'-GTCGTATCCAGTGCAGGGTCCGAGGTATTCGCACTGGATACGA   CCGGATT- 3' (SEQ ID NO: 7) |
| mRELA RT Forward | 5'- CGCATGCGCTTCTCTTCA -3' (SEQ ID NO: 8) |
| mRELA RT Reverse | 5'- GTGCAGGGTCCGAGGT -3' (SEQ ID NO: 9) |
| mRELA probe | 5'- [FAM] CGCACTGGATACGACCGGATTG (SEQ ID NO: 10) [i- |
| | EBQ] [Octamine] -3' |

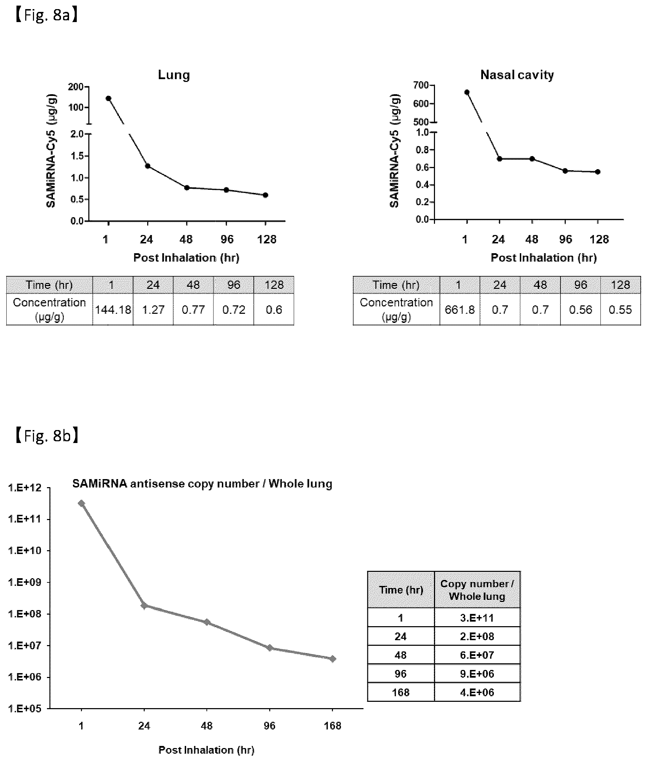

As a result, it was confirmed that the amount of SAMiRNA-Cy5 detected in the lungs harvested after administration was 144.18 µg/g (1.3%) at 1 hour after administration, 1.27 µg/g (0.01%) at 24 hours, and 0.77 µg/g (0.006%) at 48 hours. The amount of SAMiRNA-Cy5 detected in the nasal passages was 661.8 µg/g (8.2%) at 1 hour after administration, and 0.7 µg/g (0.01%) at 24 and 48 hours. It was confirmed that the SAMiRNA delivered to the nasal cavity and lung tissues by the ultrasonic nebulizer was detected in the largest amount at 1 hour after administration, and the amount of SAMiRNA detected gradually decreased over time (FIG. 8a). In the results of PK analysis of the lung tissue, similar to the results of fluorescence quantitative analysis of lung tissue, it was confirmed that the antisense copy number of SAMiRNA was the highest (3.E+11) at 1 hour after administration, 2.E+08 at 24 hours, and 6.E+07 at 48 hours, indicating that the antisense copy number of SAMiRNA gradually decreased over time (FIG. 8b)

For SAMiRNA targeting SARS-CoV-2, whether the SAMiRNA is delivered to the nasal cavity and lung tissues can be checked in the same manner as described above. SARS-CoV-2 targeting sequences are shown in Table 5 below.

**[Table 5]**

| | |
|---|---|
| SCV2-1 sense (DNA) | 5'- AATAGAGCTCGCACCGTAG -3' (SEQ ID NO: 11) |
| SCV2-1 antisense (RNA) | 5'- CUACGGUGCGAGCUCUAUU -3' (SEQ ID NO: 12) |
| SCV2-2 sense (DNA) | 5'- TGGTACTGGTAAGAGTCAT -3' (SEQ ID NO: 13) |
| SCV2-2 antisense (RNA) | 5'- AUGACUCUUACCAGUACCA -3' (SEQ ID NO: 14) |
| SCV2-3 sense (DNA) | 5'- GTTTATCACCCGCGAAGAA -3' (SEQ ID NO: 15) |
| SCV2-3 antisense (RNA) | 5'- UUCUUCGCGGGUGAUAAAC -3' (SEQ ID NO: 16) |
| SCV2-4 sense (DNA) | 5'- CATAACAGATGCGCAAACA -3' (SEQ ID NO: 17) |
| SCV2-4 antisense (RNA) | 5'- UGUUUGCGCAUCUGUUAUG -3' (SEQ ID NO: 18) |
| SCV2-5 sense (DNA) | 5'- ATAATGATGAATGTCGCAA -3' (SEQ ID NO: 19) |
| SCV2-5 antisense (RNA) | 5'- UUGCGACAUUCAUCAUUAU -3' (SEQ ID NO: 20) |
| SCV2-6 sense (DNA) | 5'- TTAAAACCAACACTACCAC -3' (SEQ ID NO: 21) |
| SCV2-6 antisense (RNA) | 5'- GUGGUAGUGUUGGUUUUAA -3' (SEQ ID NO: 22) |
| SCV2-7 sense (DNA) | 5'- ATAGTAGGGATGACATTAC -3' (SEQ ID NO: 23) |
| SCV2-7 antisense (RNA) | 5'- GUAAUGUCAUCCCUACUAU -3' (SEQ ID NO: 24) |
| SCV2-8 sense (DNA) | 5'- TCTCTATCAGACATTATGC -3' (SEQ ID NO: 25) |
| SCV2-8 antisense (RNA) | 5'- GCAUAAUGUCUGAUAGAGA -3' (SEQ ID NO: 26) |
| SCV2-9 sense (DNA) | 5'- GCTTCTTCGCGGGTGATAA -3' (SEQ ID NO: 27) |
| SCV2-9 antisense (RNA) | 5'- UUAUCACCCGCGAAGAAGC -3' (SEQ ID NO: 28) |
| SCV2-10 sense (DNA) | 5'- CCATCCGAAAGGGAGTGAG -3' (SEQ ID NO: 29) |
| SCV2-10 antisense (RNA) | 5'- CUCACUCCCUUUCGGAUGG -3' (SEQ ID NO: 30) |

### 6-3. Analysis of lung tissue biodistribution of SAMiRNA nanoparticles administered by ultrasonic nebulizer in mouse animal model

Immunofluorescence staining was performed on the lung tissue harvested from the mice exposed to each of PBS and SAMiRNA-Cy5 in Example 6-4 by the ultrasonic nebulizer. The lung tissue was stored for one day in 10% neutral buffered formalin (Sigma, US) for fixation, and then dehydrated sequentially in 10%, 20%, and 30% sucrose (Sigma, US) solutions. Each lung tissue sample was separated into left and right lobes, and then placed in a base mold (Thermo Scientific, US) containing OCT compound (Sakura Finetek, US), and a stainless plate was placed in a container containing liquid nitrogen, and the base mold was placed thereon to completely freeze the OCT compound. The frozen tissue was stored at -70°C and placed at -20°C for 30 min before microtome sectioning to facilitate tissue sectioning. The tissue sections with a thickness of 8 um were placed on a slide and dried for 1 hour. Next, the tissue was incubated for 10 minutes with 0.1% Triton-X100 (Sigma, US) solution for cell permeabilization, and then blocked with a solution containing 5% normal goat serum (Abcam, GB) and 1% BSA (Sigma, US) for 1 hour. Next, the tissue was incubated with 1 µM DAPI (Sigma, US) solution for 10 minutes and washed, and then a mounting solution (Thermo Scientific, US) was dropped thereon and covered with a cover glass (VWR, US). For fluorescence analysis of the stained tissue, the stained tissue was analyzed using spinning disk confocal microscopy (Dragonfly high-speed confocal image platform, Andor, GB). In order to accurately analyze the distribution of SAMiRNA in the lung tissue over time, confocal microscopy was performed on all the lung tissue samples under the same conditions (fluorescence intensity, exposure time, etc.).

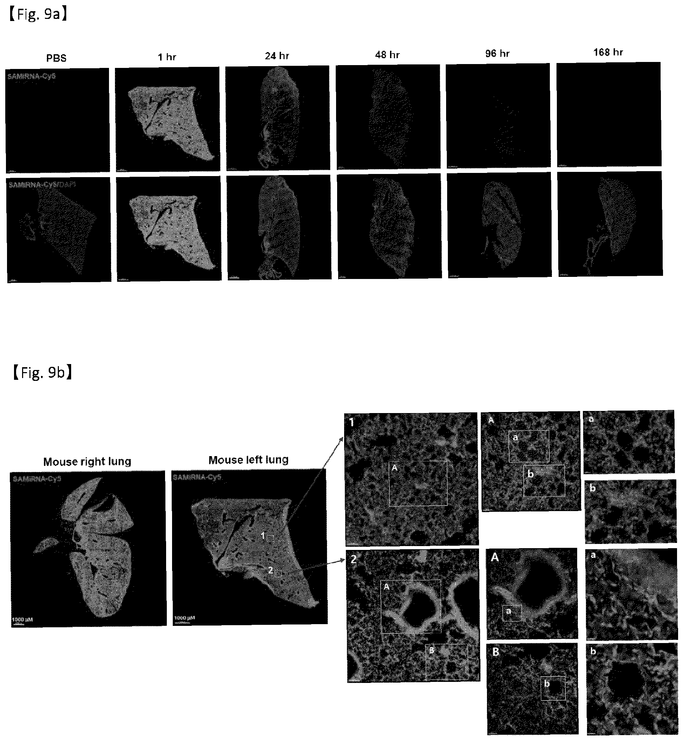

As a result, it was observed that the SAMiRNA-Cy5 fluorescence was the highest at 1 hour after administration and gradually decreased over time, similar to the results of fluorescence quantitative analysis and PK analysis of the lung tissue. It was observed that SAMiRNA was evenly distributed throughout the lung tissue for 1 hour, 24 hours, and 48 hours at which fluorescence was observed in the lung tissue (FIG. 9a). As a result of observing the left and right lung tissues 1 hour after administration, it was confirmed that SAMiRNA was evenly distributed throughout the left and right lungs, indicating that it was effectively delivered (FIG. 9b). In addition, as a result of observing the lung tissue 1 hour after administration at high magnification, it was confirmed that SAMiRNA-Cy5 was found in most cells constituting the lung tissue, and was evenly distributed in bronchioles, bronchiolar epithelial cells, alveolar epithelial cells, alveolar sacs, etc., indicating that it was effectively delivered (FIG. 9b).

### Industrial Applicability

The double-stranded oligonucleotide structure according to the present invention forms self-assembled nanoparticles, which are 90 nm in size and have a neutral charge, in an aqueous solution, and thus it may act as an optimized siRNA platform for a nebulizer from which it is nebulized in the form of fine particles of 5 um or less. In particular, when an ultrasonic nebulizer is used, it is possible to effectively deliver the pharmaceutical composition to the nasal cavity and lungs (alveolar alveoli), without changes that may occur during administration, by maintaining not only the same concentration, molecular weight, purity, nanoparticle size, and osmolality of the stock material to be administered but also the target gene inhibitory activity.

Therefore, the present invention has advantages in that it is suitable for the treatment of respiratory viral infections, pulmonary fibrosis or pneumonia caused by viral infection, or other respiratory diseases, and in particular, when the present invention is applied to a therapeutic agent for COVID-19 caused by SARS-CoV-2, a novel respiratory virus that causes explosive infections and deaths worldwide, it allows the patient to self-administer the double-stranded oligonucleotide structure easily and conveniently and enables efficient *in vivo* delivery of the double-stranded oligonucleotide structure.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Sequence List Free Text

Electronic file attached

## Claims

1. A pharmaceutical composition for preventing or treating respiratory viral infection, pulmonary fibrosis caused by viral infection, or respiratory disease comprising a double-stranded oligonucleotide structure comprising a structure represented by the following Structural Formula 1, wherein the pharmaceutical composition is administered using a nebulizer:
[Structural Formula 1] **A-X-R-Y-B**
wherein A represents a hydrophilic compound, B represents a hydrophobic compound, X and Y each independently represent a simple covalent bond or a linker-mediated covalent bond, and R represents a double-stranded oligonucleotide.

2. The pharmaceutical composition according to claim 1, wherein the nebulizer is an ultrasonic nebulizer.

3. The pharmaceutical composition according to claim 1, wherein the double-stranded oligonucleotide comprises a sense strand and an anti-sense strand comprising a sequence complementary thereto.

4. The pharmaceutical composition according to claim 3, wherein the sense strand or the antisense strand consists of 19 to 31 nucleotides.

5. The pharmaceutical composition according to claim 1, wherein the sense or antisense strand is independently DNA or RNA.

6. The pharmaceutical composition according to claim 3, wherein the sense strand or the antisense strand comprise a chemical modification.

7. The pharmaceutical composition according to claim 6, wherein the chemical modification is any one or more selected from the group consisting of:
modification in which an OH group at the 2' carbon position of a sugar structure in one or more nucleotides is substituted with any one selected from the group consisting of a methyl group (-CH₃), a methoxy group (-OCH₃), an amine group (-NH₂), fluorine (-F), a -0-2-methoxyethyl group, an -O-propyl group, an -O-2-methylthioethyl group, an -O-3-aminopropyl group, an -O-3-dimethylaminopropyl group, an -O-N-methylacetamido group, and an -O-dimethylamidooxyethyl group;
modification in which oxygen in a sugar structure in nucleotides is substituted with sulfur;
modification of a bond between nucleotides into any one bond selected from the group consisting of a phosphorothioate bond, a boranophosphate bond and a methyl phosphonate bond; and
modification to PNA (peptide nucleic acid), LNA (locked nucleic acid) or UNA (unlocked nucleic acid).

8. The pharmaceutical composition according to claim 3, wherein at least one phosphate group is bound to the 5' end of the antisense strand.

9. The pharmaceutical composition according to claim 1, wherein the double-stranded oligonucleotide is siRNA, shRNA or miRNA.

10. The pharmaceutical composition according to claim 1, comprising a structure represented by the following Structural Formula 2: 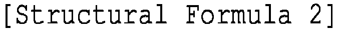 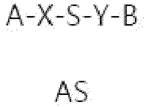 wherein S and AS respectively represent the sense strand and the antisense strand of the double-stranded oligonucleotide, and A, B, X and Y are as defined in claim 1.

11. The pharmaceutical composition according to claim 10, comprising a structure represented by the following Structural Formula 3: 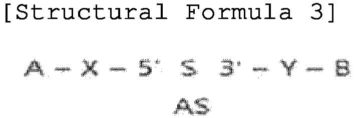 wherein A, B, X, Y, S and AS are as defined in claim 10, and 5' and 3' respectively represent the 5' and 3' ends of the sense strand.

12. The pharmaceutical composition according to claim 1, wherein the hydrophilic compound has a structure represented by the following Structural Formula 4 or Structural Formula 5:
[Structural Formula 4] (A'ₘ-J)ₙ
[Structural Formula 5] (J-A'ₘ)ₙ
wherein A' represents a hydrophilic monomer, J represents a linker that connects m hydrophilic monomers together or connects m hydrophilic monomers with siRNA, m is an integer ranging from 1 to 15, and n is an integer ranging from 1 to 10, hydrophilic monomer A' is any one compound selected from the following Compounds (1) to (3), and the linker (J) is selected from the group consisting of PO₃⁻, SO₃ and CO_{2:} 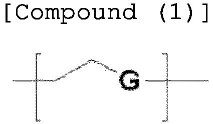
wherein G is selected from the group consisting of CH₂, O, S and NH;

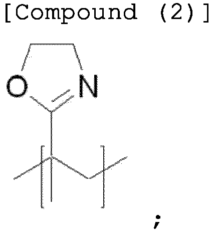

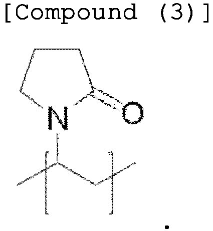

13. The pharmaceutical composition according to claim 1, wherein the hydrophilic compound has a molecular weight of 200 to 10,000.

14. The pharmaceutical composition according to claim 1, wherein the hydrophilic compound is any one selected from the group consisting of polyethylene glycol (PEG), polyvinylpyrrolidone, and polyoxazoline.

15. The pharmaceutical composition according to claim 1, wherein the hydrophobic compound has a molecular weight of 250 to 1,000.

16. The pharmaceutical composition according to claim 1, wherein the hydrophobic compound is any one selected from the group consisting of a steroid derivative, a glyceride derivative, glycerol ether, polypropylene glycol, a C₁₂-C₅₀ unsaturated or saturated hydrocarbon, diacylphosphatidylcholine, a fatty acid, a phospholipid, lipopolyamine, a lipid, tocopherol, and tocotrienol.

17. The pharmaceutical composition according to claim 16, wherein the steroid derivative is any one selected from the group consisting of cholesterol, cholestanol, cholic acid, cholesteryl formate, cholestanyl formate, and cholestanyl amine.

18. The pharmaceutical composition according to claim 16, wherein the glyceride derivative is any one selected from the group consisting of mono-glyceride, di-glyceride, and triglyceride.

19. The pharmaceutical composition according to claim 1, wherein the covalent bond represented by each of X and Y is either a nondegradable bond or a degradable bond.

20. The pharmaceutical composition according to claim 19, wherein the nondegradable bond is an amide bond or a phosphate bond.

21. The pharmaceutical composition according to claim 19, wherein the degradable bond is any one selected from the group consisting of a disulfide bond, an acid-degradable bond, an ester bond, an anhydride bond, a biodegradable bond, and an enzyme-degradable bond.

22. The pharmaceutical composition according to claim 1, wherein the respiratory disease is selected from the group consisting of interstitial lung disease, chronic obstructive pulmonary disease (COPD), pneumonia, asthma, acute and chronic bronchitis, allergic rhinitis, bronchitis, bronchiolitis, pharyngitis, tonsillitis, and laryngitis.

23. The pharmaceutical composition according to claim 1, wherein the double-stranded oligonucleotide specifically inhibits expression of a gene selected from the group consisting of amphiregulin, RelA/p65, and SARS-CoV-2.

24. The pharmaceutical composition according to claim 1, wherein the double-stranded oligonucleotide structure forms self-assembled nanoparticles, which are 10 to 100 nm in size and have a neutral charge, in an aqueous solution for administration.

25. The pharmaceutical composition according to claim 24, wherein the nanoparticle is composed of a mixture of double-stranded oligonucleotide structures comprising double-stranded oligonucleotides comprising different sequences.
